(19) Europäisches Patentamt
European Patent Office
Office européen des brevets

(11) Veröffentlichungsnummer: **0 183 091**
**A2**

# (12) EUROPÄISCHE PATENTANMELDUNG

(21) Anmeldenummer: **85114017.8**

(22) Anmeldetag: **04.11.85**

(51) Int. Cl.⁴: **C 07 D 413/04**
C 07 D 413/14, C 07 D 417/04
A 61 K 31/44

(30) Priorität: **27.11.84 DE 3443179**

(43) Veröffentlichungstag der Anmeldung:
**04.06.86 Patentblatt 86/23**

(84) Benannte Vertragsstaaten:
**AT BE CH DE FR GB IT LI LU NL SE**

(71) Anmelder: **CASSELLA Aktiengesellschaft**
**Hanauer Landstrasse 526**
**D-6000 Frankfurt am Main 61(DE)**

(72) Erfinder: **Schönafinger, Karl, Dr.**
**104 Arapaho Trail**
**Somerville New Jersey 08876(US)**

(72) Erfinder: **Bohn, Helmut, Dr.**
**Kranzbergring 11**
**D-6369 Schöneck(DE)**

(72) Erfinder: **Martorana, Piero, Dr.**
**Kaiser Friedrich Promenade 108**
**D-6380 Bad Homburg(DE)**

(72) Erfinder: **Nitz, Rolf-Eberhard, Dr.**
**Heinrich Bingemer Weg 64**
**D-6000 Frankfurt am Main 60(DE)**

(74) Vertreter: **Urbach, Hans-Georg, Dr. et al,**
**Hanauer Landstrasse 526**
**D-6000 Frankfurt am Main 61(DE)**

## (54) 1,4-Dihydropyridine, Verfahren zu ihrer Herstellung und ihre Verwendung.

(57) 1, 4-Dihydropyridine der Formel I

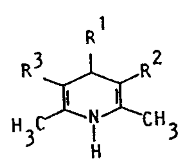

(I)

worin
$R^1$ gegebenenfalls substituiertes Phenyl, Pyridyl oder Thienyl,
$R^2$ gegebenenfalls substituiertes Oxadiazolyl, Thiadiazolyl oder Thiazolyl und
$R^3$ Wasserstoff oder -COOH bedeutet,
ihre Herstellung durch modifizierte Hantzsch-Synthese und ihre Verwendung als Calziumagonist in der Pharmazie.

EP 0 183 091 A2

# 1,4-Dihydropyridine, Verfahren zu ihrer Herstellung und ihre Verwendung

Die Erfindung betrifft 1,4-Dihydropyridine der Formel I

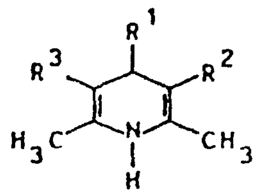

(I)

worin

$R^1$ Phenyl, Pyridyl oder Thienyl,

$R^2$ Oxadiazolyl, Thiadiazolyl oder Thiazolyl und

$R^3$ Wasserstoff oder -COOH bedeutet,

wobei ein für $R^1$ stehender Phenyl-, Pyridyl- oder Thienyl-Rest unsubstituiert ist oder einen oder zwei gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyan, Benzyloxy aufweist, ein für $R^2$ stehender Oxadiazolyl-, Thiadiazolyl- oder Thiazolyl-Rest unsubstituiert ist oder einen, oder im Falle von Thiazolyl einen oder zwei gleiche oder verschiedene, Substituenten aus der Gruppe Alkyl mit 1 bis 4 C-Atomen, Alkoxy mit 1 bis 4 C-Atomen, Alkylthio mit 1 bis 4 C-Atomen, Aralkyl mit 7 bis 9 C-Atomen, Alkoxyalkyl mit insgesamt 2 bis 5 C-Atomen, Cycloalkyl mit 5 oder 6 C-Atomen, Aminocarbonyl-methylthio, Methoxycarbonyl, Ethoxycarbonyl oder Phenyl aufweist, deren Salze mit pharmakologisch akzeptablen Säuren sowie die Herstellung dieser Verbindungen und ihre Verwendung als Arzneimittel.

Für $R^1$ stehende Pyridylreste sind Pyrid-2-yl, Pyrid-3-yl und Pyrid-4-yl; für $R^2$ stehende Thienylreste sind Thien-2-yl oder Thien-3-yl.

Von den für $R^2$ stehenden Thiazolyl-, Thiadiazolyl- und Oxadiazolylresten sind 1,3-Thiazol-2-yl, 1,2,4-Thiadiazol-5-yl, 1,3,4-Thiadiazol-2-yl, 1,2,4-Oxadiazol-5-yl und 1,2,4-Oxadiazol-2-yl bevorzugt.

Für $R^1$ stehende substituierte Phenylreste sind in 2-, 3- oder 4-Position, vorzugsweise in 2- oder 3-Position, oder in 2- und 3-Position substituiert.

Alkylreste und Alkoxyreste, die gegebenenfalls als Substituenten an einen für $R^1$ oder $R^2$ stehenden Phenylrest oder heterocyclischen Rest gebunden sind, können geradkettig oder verzweigt sein. Beispiele für solche Reste sind Methyl, Ethyl, Propyl-(1), Propyl-(2), n-Butyl-(1), n-Butyl-(2), 2-Methylpropyl-(1), tert.Butyl, Methoxy, Ethoxy, Prop-1-oxy, Prop-2-oxy, n-Butoxy, But-2-oxy, 2-Methyl-prop-1-oxy, tert.Butoxy.

Bevorzugt sind Alkyl- und Alkoxyreste mit 1 oder 2 C-Atomen, insbesondere Methyl und Methoxy.

Ein als Substituent eines für $R^2$ stehenden heterocyclischen Restes stehender Alkylthiorest kann ebenfalls geradkettig oder verzweigt sein. Beispiele für solche Alkylthioreste sind Methylthio, Ethylthio, Prop-1-ylthio, Prop-2-ylthio, n-But-1-ylthio, But-2-ylthio, 2-Methyl-prop-1-ylthio, tert.Butylthio. Bevorzugt sind Alkylthioreste mit 1 oder 2 C-Atomen, insbesondere Methylthio. Aralkylreste, die gegebenenfalls Substituent eines für $R^2$ stehenden Heterocyclus sind, sind insbesondere Phenalkylreste mit 1 bis 3 C-Atomen in der Alkylbrücke, wie Benzyl, Phenethyl, 3-Phenylprop-1-yl, 2-Phenylprop-1-yl, 1-Phenylprop-2-yl und 2-Phenylprop-2-yl. Bevorzugte Phenalkylreste sind Benzyl und Phenethyl.

Auch die Kohlenstoffketten von Alkoxyalkylresten, die gegebenenfalls Substituenten eines für $R^2$ stehenden Heterocyclus sind, können geradkettig oder verzweigt sein.

Beispiele für solche Alkoxyalkylreste sind Methoxymethyl, Ethoxymethyl, Prop-1-oxymethyl, Prop-2-oxymethyl, n-Butoxymethyl, sec.Butoxymethyl, Isobutoxymethyl, tert.-Butoxymethyl, Methoxyethyl, Ethoxyethyl, Propoxyethyl, Isopropoxyethyl, 1-Methoxy-propyl-(2), 1-Methoxy-propyl-(3), 2-Methoxy-propyl-(1), die entsprechenden, isomeren Ethoxypropylreste, die 12 isomeren Methoxybutylreste.

Bevorzugt sind solche Alkoxyalkylreste, die unverzweigte und über primäre C-Atome gebundene Kohlenstoffketten enthalten.

Von den als mögliche Substituenten eines für $R^2$ stehenden Heterocyclus genannten Cycloalkylresten ist der Cyclohexylrest bevorzugt.

Halogenatome, die gegebenenfalls Substituenten eines für $R^1$ stehenden Phenylrestes sind, sind vorzugsweise Fluor, Chlor oder Brom, insbesondere Chlor oder Brom; besonders bevorzugtes Halogen ist Chlor.

So ist eine bevorzugte Bedeutung von $R^1$ Phenyl, das unsubstituiert ist oder das einen oder zwei gleiche oder verschiedene Substituenten aus der Reihe Halogen, Methyl, Methoxy, Nitro, Trifluormethyl, Cyan oder Benzyloxy aufweist.

Beispiele für einen für $R^1$ stehenden substituierten Phenylrest sind: 2-Chlorphenyl, 3-Chlorphenyl, 4-Chlorphenyl, 2,3-Dichlorphenyl, 2-Nitrophenyl, 3-Nitrophenyl, 3-Cyanophenyl, 3-Methoxyphenyl, 3-Trifluormethylphenyl, 2-Trifluormethylphenyl, o-Tolyl, m-Tolyl oder p-Tolyl. Besonders bevorzugt bedeutet $R^1$ ein durch Cyan, Nitro oder Chlor monosubstituiertes oder durch Chlor disubstituiertes Phenyl, wobei die Substituenten vorzugsweise in 2- und/oder 3-Stellung des Phenylkerns vorhanden sind.

Ganz besonders bevorzugt bedeutet $R^1$ 2-Nitrophenyl, 3-Nitrophenyl, 3-Cyanophenyl, 2-Chlorophenyl und 2,3-Dichlorophenyl.

Besonders bevorzugte Substituenten für einen für $R^2$ stehenden Heterocyclus, insbesondere für einen 1,2,4-oder 1,3,4-Oxadiazolylrest, sind: Methyl, Ethyl, Isopropyl und Benzyl. Besonders bevorzugt für $R^2$ sind 1,3,4-Oxadiazol-2-yl, 5-Methyl-1,3,4-oxadiazol-2-yl, 5-Ethyl-1,3,4-oxadiazol-2-yl, 3-Methyl-1,2,4-oxadiazol-5-yl, 3-Ethyl-1,2,4-oxadiazol-5-yl, 3-Benzyl-1,2,4-oxadiazol-5-yl.

Die Herstellung der erfindungsgemäßen Verbindungen der Formel I erfolgt nach Analogieverfahren, die Varianten der bekannten Hantzsch-Synthese darstellen. Der zweckmäßigste Syntheseweg zum Aufbau des Dihydropyridin-Systems steht in der Kondensation von 1 mol eines leicht hydrolisierbaren 3-Aminocrotonsäureesters, vorzugsweise des 3-Aminocrotonsäure-ß-cyanethyl-esters (Formel IIa) mit 1 mol einer Ylidenverbindung, dem α,ß-ungesättigten Keton der Formel III:

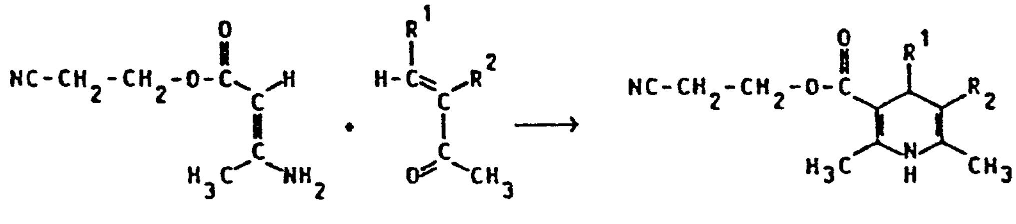

(IIa) (III) (Ia)

Nach der Synthese des Dihydropyridin-carbonsäureesters der Formel Ia wird die Esterfunktion in an sich bekannter Weise im wäßrigen Medium in Gegenwart einer Säure oder, vorzugsweise, einer Base verseift. Hierbei werden dann die erfindungsgemäßen Verbindungen der Formel I erhalten, in denen $R^3$ die Carboxylgruppe (-COOH) bzw. deren Salz ist.

Sollen erfindungsgemäße Verbindungen der Formel I hergestellt werden, in denen $R^3$ Wasserstoff ist, so werden die entsprechenden Verbindungen, in denen $R^3$ die Carboxylgruppe bedeutet, in an sich bekannter Weise decarboxyliert.

Die vorliegende Erfindung betrifft somit auch ein Verfahren zur Herstellung der 1,4-Dihydropyridine der Formel I,

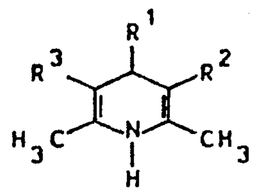

(I)

worin $R^1$, $R^2$ und $R^3$ die oben angegebenen Bedeutungen haben, das dadurch gekennzeichnet ist, daß man einen Aminocrotonsäureester der Formel II

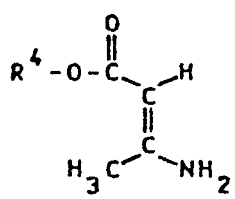

(II)

worin $R^4$ der Rest eines hydrolytisch leicht abspaltbaren Alkohols ist, mit einem α,β-ungesättigten Keton der Formel III

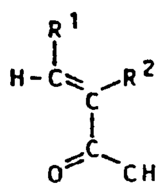

(III)

worin $R^1$ und $R^2$ die oben genannten Bedeutungen haben, in einem inerten organischen Lösungsmittel bei einer Temperatur zwischen 10°C und dem Siedepunkt des eingesetzten Lösungsmittels zu einem 1,4-Dihydropyridin-carbonsäureester der Formel Ia

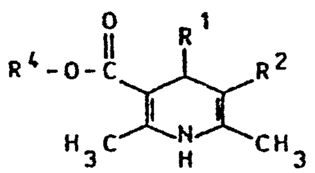

(Ia)

worin $R^1$ bis $R^4$ die oben genannten Bedeutungen haben, umsetzt, anschließend diesen Ester in wäßrigem Medium in Gegenwart einer Säure oder einer Base zu einer Verbindung der Formel I, in der $R^3$ die Carboxylgruppe ist, hydrolisiert und gewünschtenfalls die erhaltene Verbindung in an sich bekannter Weise decarboxyliert und gegebenenfalls mit einer pharmakologisch akzeptablen Säure zu dem entsprechenden Säureadditionssalz umsetzt.

Reste $R^4$, die sich hydrolytisch leicht abspalten lassen, leiten sich von Alkoholen ab, die in Konjugation oder in Nachbarposition zur Hydroxylgruppe einen elektronenanziehenden Substituenten tragen. Beispiele für elektronenanziehende Substituenten sind z.B. die Carbonylgruppe, die Sulfonylgruppe, die Sulfoxidgruppe, Carbon- oder Sulfonestergruppen, die Nitro- und die Cyangruppe. Besonders bevorzugt für $R^4$ ist der ß-Cyanethylrest.

3-Aminocrotonsäureester der Formel II sind bekannte Verbindungen. Sie können leicht durch Umsetzung des entsprechenden Acetessigesters mit Ammoniak hergestellt werden. Auch der besonders bevorzugte 3-Aminocrotonsäure-ß-cyanethylester läßt sich analog leicht, z.B. gemäß den

Angaben in DE-OS 21 17 571 und DE-OS 21 17 572 herstellen.

Die als Ausgangskomponenten benötigten Yliden-Verbindungen der Formel III können, soweit sie nicht bereits bekannt sind, hergestellt werden nach Org.Reactions XV, 204 ff, (1967). Beispiele für geeignete Ausgangsverbindungen der Formel III sind: 1-(2,3-Dichlorphenyl)-2-(1,3,4-oxadiazol-2-yl)-1-buten-3-on, 1-(3-Nitrophenyl)-2-(1,3,4-oxadiazol-2-yl)-1-buten-3-on, 1-(2-Nitrophenyl)-2-(1,3,4-oxadiazol-2-yl)-1-buten-3-on, 1-(2-Chlorphenyl)-2-(1,3,4-oxadiazol-2-yl)-1-buten-3-on, 1-(3-Cyanophenyl)-2-(1,3,4-oxadiazol-2-yl)-1-buten-3-on, 1-Pyridin-3-yl)-2-(1,3,4-oxadiazol-2-yl)-1-buten-3-on, 1-(Thien-2-yl)-2-(1,3,4-oxadiazol-2-yl)-1-buten-3-on, 1-(3-Trifluormethyl-phenyl)-2-(1,3,4-oxadiazol-2-yl)-1-buten-3-on, 1-(2,3-Dichlorphenyl)-2-(2-methyl-1,3,4-oxadiazol-5-yl)-1-buten-3-on, 1-(2,3-Dichlorphenyl)-2-(2-benzyl-1,3,4-oxadiazol--5-yl)-1-buten-3-on, 1-(2-Methylphenyl)-2-(3-ethyl-1,2,4-oxadiazol-5-yl)-1-buten-3-on, 1-(3-Methoxyphenyl)-2-(3-tert.butyl-1,3,4-oxadiazol-5-yl)-1-buten-3-on, 1-(3-Chlorphenyl)-2-(1,2,4-thiadiazol-5-yl)-1-buten-3-on, 1-(2-Trifluormethyl)-2-(3-benzyl-1,2,4-oxadiazol-5-yl)-1-buten-3-on, 1-(2,5-Dichlorphenyl)-2-(3-methylthio--1,3,4-oxadiazol)-1-buten-3-on, 1-(Pyridin-2-yl)-2-(4-methyl-5-ethoxycarbonyl-thiazol-2-yl)-1-buten-3-on, 1-(3-Nitrophenyl)-2-(3-methyl-1,2,4-oxadiazol-5-yl)-1-buten-3-on, 1-(2,3-Dichlorphenyl)-2-(3-ethyl-1,2,4-oxadiazol-5-yl)-1-buten-3-on.

Eine besonders zweckmäßige Ausführungsform dieses Herstellungsverfahrens besteht darin, daß die Kondensation des Aminocrotonsäureesters mit der Yliden-Verbindung kombiniert wird mit der Synthese der Yliden-Verbindung in einem Eintopf-Prozeß.

Hierbei wird die Yliden-Verbindung durch Kondensation eines Aldehyds der Formel IV mit einem Keton der Formel V gewonnen. Bei dieser Verfahrensvariante wird somit zum Aufbau des Dihydropyridingerüstes 1 mol des leicht hydrolisierbaren 3-Aminocrotonsäureesters mit je 1 mol eines Aldehyds der Formel IV und eines Ketons der Formel V umgesetzt.

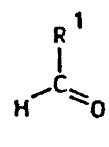

(IV)

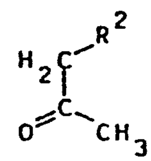

(V)

Es ist für den Fachmann eine Selbstverständlichkeit, daß bei der Kondensation des Aminocrotonsäureesters der Formel II mit dem ungesättigten Keton der Formel III oder mit dem Aldehyd der Formel IV und dem Keton der Formel V die eine oder andere Komponente in einem Überschuß bis zu 100 %, vorzugsweise bis zu 20 %, eingesetzt werden kann, wenn dies im Interesse der vollständigen Umsetzung einer der Komponenten, z.B. einer schwer zugänglichen und daher entsprechend teuren Komponente, zweckmäßig erscheint oder wenn die im Überschuß eingesetzte Komponente besonders leicht vom Endprodukt getrennt werden kann. Es ist dem Fachmann auch bekannt, daß der Einsatz eines Überschusses einer der Komponenten zu einer Steigerung der Reaktionsgeschwindigkeit unter ansonsten konstanten Reaktionsbedingungen führt bzw. die Ausführung der Reaktion unter milderen Bedingungen gestattet.

Die bei dieser Verfahrensvariante als Ausgangskomponenten dienenden Aldehyde der Formel IV können, falls sie nicht bereits bekannt sind, z.B. hergestellt werden nach den von E. Mosettig, Org. Reactions VIII, 218 ff. (1954) beschriebenen Methoden. Beispiele für geeignete Aldehyde

der Formel IV sind: Benzaldehyd, 2-, 3- oder 4-Methylbenzaldehyd, 2-, 3- oder 4-Ethyl-benzaldehyd, 2-, 3- oder 4-i-Propyl-benzaldehyd, 2-, 3- oder 4-tert.-Butyl-benzaldehyd, 2-, 3- oder 4-Methoxy-benzaldehyd, 2-, 3- oder 4-i-Propoxy-benzaldehyd, 2-, 3- oder 4-Brom-benzaldehyd, 2-, 3- oder 4-Chlor-benzaldehyd, 2-, 3- oder 4-Fluorbenzaldehyd, 2-, 3- oder 4-Cyano-benzaldehyd, 2-, 3- oder 4-Trifluormethyl-benzaldehyd, 2-, 3- oder 4-Nitro-benzaldehyd, 2,4- oder 2,6-Dimethyl-benzaldehyd, 2,4-oder 2,6-Dichlor-benzaldehyd, 2,4- oder 2,6-Dibrom-benzaldehyd, 2,4- oder 2,6-Dinitrobenzaldehyd, 2,4- oder 2,6-Diethylbenzaldehyd, 3-Chlor-4-trifluormethyl-benzaldehyd, 3-Methyl-4-trifluormethyl-benzaldehyd, 3-Methoxy-4-chlor-benzaldehyd, 2-Methyl-4-cyano-benzaldehyd, Pyridin-2-aldehyd, Pyridin-3-aldehyd, Pyridin-4-aldehyd, 4-Methyl-pyridin-2-aldehyd, 5-Methylpyridin-2-aldehyd, 6-Methyl-pyridin-2-aldehyd, Thiophen2-aldehyd, Thiophen3-aldehyd, 5-Nitro-thiophen-2-aldehyd, 5-Methyl-thiophen-2-aldehyd, 5-Chlor-thiophen-2-aldehyd, 5-Methoxy-thiophen-2-aldehyd.

Die Ketone der Formel V können, soweit sie nicht bereits bekannt sind, nach dem in Monatshefte für Chemie 113, 781 ff. (1982) beschriebenen Verfahren hergestellt werden. Geeignete Ausgangsverbindungen der Formel V sind z.B. 5-Acetonyl-1,2,4-oxadiazol, 3-Methyl-5-acetyl-1,2,4-oxa-diazol, 3-Ethyl-5-acetyl-1,2,4-oxadiazol, 3-tert.Butyl-5-acetyl-1,2,4-oxadiazol, 3-Methylthio-5-acetyl-1,2,4-oxadiazol, 3-Benzyl-5-acetyl-1,2,4-oxadiazol, 2-Aceto-nyl-1,3,4-oxadiazol, 5-Methyl-2-acetonyl-1,3,4-oxadiazol, 5-i-Propyl-2-acetonyl-1,3,4-oxadiazol, 3-Acetonyl-1,2,4-oxadiazol, 5-Ethyl-3-acetonyl-1,2,4-oxadiazol, 5-Ethylthio-3-acetonyl-1,2,4-oxadiazol, 5-Phenethyl-3-acetonyl-1,2,4-oxadiazol, 5-Acetonyl-1,2,4-thiadiazol, 3-Ethyl-5-acetonyl-1,2,4-thiadiazol, 3-Benzyl-5-acetonyl-1,2,4-thiadiazol.

Der Aufbau des Dihydropyridingerüstes nach den beiden angegebenen Verfahrens-Varianten wird bei Raumtemperatur (20°C) oder insbesondere erhöhter Temperatur, z.B. in einem Bereich von 20 bis 120°C, durchgeführt. Vorzugsweise wird die Umsetzung bei beiden Varianten im Temperaturbereich von 40 bis 100°C, insbesondere bei der Rückflußtemperatur des verwendeten Lösungsmittels oder Lösungsmittelgemischs, durchgeführt. Normalerweise erfolgt die Umsetzung bei Normaldruck, kann aber auch bei einem vom Normaldruck abweichenden Druck durchgeführt werden.

Die Umsetzungen werden in Wasser oder einem inerten organischen Lösungsmittel durchgeführt. Geeignete Lösungsmittel sind z.B. Alkohole, insbesondere solche mit 1 bis 6 C-Atomen, wie z.B. Methanol, Ethanol, i- und n-Propanol, i-, sec.- und tert.-Butanol, n-, i-, sec.-, tert.-Pentanol, n-Hexanol, Cyclopentanol, Cyclohexanol; Ether, insbesondere solche mit 2 bis 8 C-Atomen im Molekül, wie z.B. Diethylether, Methyl-ethyl-ether, Di-n-propyl-ether, Di-iso-propyl-ether, Methyl-n-butyl-ether, Ethylpropylether, Di-butyl-ether, Tetrahydrofuran; 1,4-Dioxan, 1,2-Dimethoxyethan, Bis-ß-methoxyethyl-ether; Polyether, wie z.B. Polyethylenglykole mit einem Molekulargewicht bis ca. 600; Oligoethylen-glykol-dimethyl-ether, wie z.B. Pentaglyme; Glykole und teilweise veretherte Glykole, wie z.B. Ethylenglykol, Propylenglykol, Trimethylenglykol, Ethylenglykol-monomethyl-ether, Ethylenglykol-monoethylether, Diethylenglykol-monoethyl-ether; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-isopropylketon, Di-iso-butylketon, Cyclopentanon, Cyclohexanon, Benzophenon, Acetophenon; aliphatische Kohlenwasserstoffe, wie z.B. niedrig- und hochsiedende Petrolether; aromatische Kohlenwasserstoffe, wie z.B. Benzol, Toluol, o-, m- und p-Xylol, Pyridin; halogenierte aliphatische oder aromatische Kohlenwasserstoffe, wie z.B. Methylenchlorid, Chloroform, Tetrachlorkohlenstoff, Ethylenchlorid, Chlorbenzol, Dichlorbenzol; Nitrile, wie z.B. Acetonitril; Amide, wie z.B. Dimethylformamid, N-Methyl-pyrrolidon; Hexamethylphosphorsäuretriamid; Sulfoxide, wie z.B. Dimethylsulfoxid; Wasser. Auch Gemische verschiedener Lösungsmittel können verwendet werden. Alkohole oder Gemische von Alkoholen mit Wasser sind in der Regel bevorzugt.

Die Verseifung der Ester der Formel Ia kann, wie bereits oben ausgeführt, durch Hydrolyse in Gegenwart von Säuren oder von Basen erfolgen. Der Einsatz von Basen ist bevorzugt. Als Basen kommen in erster Linie anorganische Basen in Betracht, vorzugsweise Alkalihydroxide, wie Natrium- oder Kaliumhydroxid. Die Basen könnnen in molaren Mengen oder in 2- bis 4-fachem Überschuß eingesetzt werden.

Als Reaktionsmedium hat sich Wasser als vorteilhaft erwiesen. Zur homogenen Reaktionsführung ist es in der Regel zweckmäßig, ein inertes, mit Wasser mischbares organisches Lösungsmittel zuzusetzen. Hierzu gehören Alkohole, wie Methanol, Ethanol, Propanol, Isopropanol, 2-Methoxyethanol, Ether, wie Dioxan, Tetrahydrofuran, 1,2-Dimethoxyethan oder Pyridin, Dimethylformamid, Dimethylsulfoxid.

Besonders vorteilhaft ist die Verwendung von Methanol, Ethanol, 2-Methoxyethanol und 1,2-Dimethoxyethan. Die Reaktionstemperaturen liegen im allgemeinen zwischen 0 bis 100°C, insbesondere zwischen 20 bis 50°C, vorzugsweise bei Raumtemperatur. Bei der Durchführung der Hydrolyse fallen die Verbindungen I als Salze in der Reaktionsmischung an. Deshalb wird die Mischung zweckmäßigerweise mit Wasser verdünnt, mit Methylenchlorid ausgeschüttelt. Die wäßrige Phase wird dann sauer gestellt, wobei die erfindungsgemäßen Verbindungen der Formel I, in

denen $R^3$ die Carboxylgruppe ist, in aller Regel ausfallen und durch Filtrieren abgetrennt werden können. Die eventuell gewünschte Decarboxylierung dieser Verbindungen zu den erfindungsgemäßen Verbindungen der Formel I, in denen $R^3$ Wasserstoff ist, wird in aller Regel bei 100 bis 200°C durchgeführt, wobei es sich als zweckmäßig herausgestellt hat, in einem in diesem Temperaturbereich siedenden inerten Lösungsmittel zu arbeiten. Derartige Lösungsmittel sind z.B. Toluol, Xylol, Chlorbenzol, Dichlorbenzol, Nitrobenzol, Glykol, Methoxyethanol, Diethylenglykol, 2-Ethoxyethanol, Propandiol, Dimethylsulfoxid; besonders bevorzugt sind Diethylenglykol und Temperaturen von 160 bis 180°C.

Die erfindungsgemäßen 1,4-Dihydropyridin-Derivate der Formel I bilden, sofern sie basische Substituenten besitzen, mit anorganischen oder organischen Säuren Säureadditionssalze. Geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar, Malein-, Apfel-, Sulfamin-, Phenylpropion-, Glucon-, Ascorbin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungs- oder Verdünnungsmittel, hergestellt. Bei der Synthese der Verbindungen der Formel I können zunächst die Säureadditionssalze im Zuge der Aufarbeitung anfallen. Aus den Säureadditionsalzen können die freien Verbindungen der allgemeinen Formel I gewünschenfalls in bekannter Weise, z.B. durch Auflösen oder Suspendieren in Wasser und Alkalischstellen, z.B. mit Natronlauge, und anschließendes Isolieren, gewonnen werden.

Die erfindungsgemäßen Verbindungen der Formel I besitzen in der 4-Stellung des Dihydropyridinrings ein asymmetrisches Kohlenstoffatom. Diese Verbindungen treten daher als Racemate und in Form der optisch aktiven Enantiomeren auf. Für den Fall, daß die Verbindungen der Formel I mehr als ein asymmetrisches Atom besitzen, treten auch Diastereomere und ihre Mischungen auf. Mischungen von Diastereomeren und racemische Mischungen von Enantiomeren lassen sich nach bekannten Verfahren in die Einzelkomponeten auftrennen. Mischungen von Diastereomeren lassen sich z.B. durch fraktionierte Umkristallisation oder mit Hilfe von chromatographischen Verfahren in die Diastereomeren auftrennen. Ein Racemat läßt sich z.B. durch Umsetzung mit einer geeigneten enantiomeren Verbindung in ein diastereomeres Salzgemisch überführen, das dann z.B. durch fraktionierte Umkristallisation in die einzelnen diastereomeren Salze aufgetrennt wird. Die diastereomeren Salze werden dann in bekannter Weise in die enantiomeren Verbindungen überführt.

Es ist bereits bekannt, daß bestimmte 1,4-Dihydropyridine interessante pharmakologische Eigenschaften aufweisen (F. Bossert, W. Vater "Die Naturwissenschaften" 58, 578, (1971). In der Regel stellen die bekannten wirksamen Verbindungen 1,4-Dihydropyridin-3,5-dicarbonester dar.

Es ist auch bereits bekannt, daß 1,4-Dihydropyridine gefäßerweiternde Eigenschaften besitzen und als Coronarmittel und Antihypertensiva verwendet werden können (vgl. z.B. DE-OS 26 29 892 und DE-OS 27 52 820). Diese Wirkung der 1,4-Dihydropyridine beruht auf einer kalciumantagonistischen Wirkung (vgl. A. Fleckenstein, Ann.Rev.Pharmacol. Toxicol. 17, 149 bis 166 (1977)).

Von 5-Oxadiazolyl-, 5-Thiazolyl- und 5-Thiadiazolyl-dihydropyridin-3-carbonestern ist ebenfalls eine kalciumantagonistische Wirkung bekannt geworden (vergl. DE-OS 32 47 118). Im Gegensatz dazu ist aus der EP-A-71 819 bekannt, daß Dihydropyridinderivate der allgemeinen Formel A

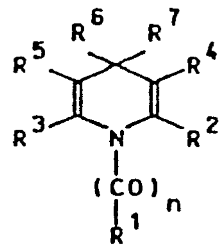

(A)

in der die Symbole $R^1$ bis $R^7$ breite Definitionsbereiche haben, eine positiv inotrope Wirkung zeigen.

Die Verbindungen der Formel I der vorliegenden Erfindung zeigen nun eine ausgeprägte calziumagonistische Wirkung und sind den in der EP-A-71 819 offenbarten Verbindungen im Hinblick auf die Wirkdauer erheblich überlegen.

Die Verbindungen der Formel I und ihre pharmakologisch annehmbaren Säureadditionssalze können daher mit besonderem Vorteil am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon neben üblichen pharmazeutisch einwandfreien Träger- und Zusatzstoffen enthalten. Die Zubereitungen enthalten normalerweise etwa 0,5 bis 90 Gewichtsprozent der therapeutisch wirksamen Verbindung.

Die Heilmittel können oral, z.B. in Form von Pillen, Tabletten, Lacktabletten, Dragees, Granulaten, Hart- und Weichgelatinekapsel, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks-oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I und/oder ihrer pharmakologisch annehmbaren Säureadditionssalze und noch andere therapeutisch wirksame Stoffe enthalten.

Derartige andere therapeutisch wirksame Substanzen sind beispielsweise: ß-Rezeptorenblocker, wie z.B. Propranolol, Pindolol, Metoprolol; antianginöse Mittel, wie z.B. Carbochromen oder Molsidomin; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin; Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Dosierung kann innerhalb weiter Grenzen variieren und ist in jedem einzelnen Fall den individuellen Gegebenheiten anzupassen. Im allgemeinen ist bei oraler Verabreichung eine Tagesdosis von etwa 0,01 bis 10 mg/kg, vorzugsweise 0,05 bis 5 mg/kg, Körpergewicht zur Erzielung wirksamer Ergebnisse angemessen. Bei intravenöser Applikation beträgt die Tagesdosis im allgemeinen etwa 0,001 bis 10 mg/kg, vorzugsweise 0,01 bis 5 mg/kg, Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es je nach individuellem Verhalten erforderlich werden, von der angegebenen Tagesdosis nach oben oder unten abzuweichen.

Zum Nachweis der hämodynamischen Wirkung der erfindungsgemäßen Verbindungen wurden Untersuchungen am Bastardhund in Pentobarbital-Narkose (30 bis 40 mg/kg i.v.) durchgeführt. Die Beatmung erfolgte mit einem Bird-Mark-7-Respirator. Der endexpiratorische Kohlensäuregehalt (gemessen mit einem Ultrarotabsorptionsschreiber) betrug zwischen 4,5 und 5 Vol.%. Während des gesamten Versuchs wurde eine Dauerinfusion von Pentobarbital i.v.= 5 mg (in 6 ml)/kg/h

durch die Vena cephalica gegeben, um eine konstante Narkosetiefe zu gewährleisten. Vor dem eigentlichen Versuch wurde ca. 1 Stunde gewartet, bis sich alle haemodynamischen Parameter eingestellt hatten (steady state). Dann wurde mit dem eigentlichen Versuch begonnen. Der systolische und diastolische Blutdruck wurde peripher in der Arteria femoralis über einen Statham-Druckaufnehmer gemessen.

Die erhaltenen Ergebnisse sind in der folgenden Tabelle angegeben:

| Blutdruck | Präparat 1 0,1 mg/kg i.v. | Präparat 2 0,1 mg/kg i.v. |
|---|---|---|
| systolisch | | |
| 1. vor der Verabreichung, mmHg | 130 | 115 |
| 2. nach der Verabreichung, mmHg | 340 | 270 |
| 3. Differenz 2-1, mmHg | 210 | 155 |
| 4. Wirkdauer, min | 10 | 5 |
| diastolisch | | |
| 1. vor der Verabreichung, mmHg | 95 | 75 |
| 2. nach der Verabreichung, mmHg | 180 | 200 |
| 3. Differenz 2-1, mmHg | 85 | 125 |
| 4. Wirkdauer, min | 10 | 5 |

Präparat 1 = 1,4-Dihydro-2,6-dimethyl-3-(1,3,4-oxadiazol-2-yl)-4-(2-chlorphenyl)-pyridin (gemäß nachfolgendem Beispiel 3)

Präparat 2 = 1,4-Dihydro-2,6-dimethyl-3-nitro-4-(2-trifluormethyl-phenyl)-pyridin-3-carbonsäuremethylester (Vergleichssubstanz).

Durch die folgenden Ausführungsbeispiele wird die Erfindung weiter veranschaulicht.

Beispiel 1:
1,4-Dihydro-2,6-dimethyl-4-(2-chlorphenyl)-5-(1,3,4-oxadiazol-2-yl)-pyridin-3-carbonsäure

18,9 g (1,3,4-Oxadiazol-2-yl)-aceton, 23 g Aminocrotonsäure-cyanethylester und 20 g 2-Chlorbenzaldehyd werden in 100 ml Isopropanol 8 h am Rückfluß gekocht. Die Mischung wird anschließend über Nacht bei Raumtemperatur und 5 h bei 0°C gerührt. Der ausgefallene Niederschlag wird abgesaugt und aus Ether/Petrolether umkristallisiert:
Ausbeute: 24 g, (-44,7 % d. Theorie) 1,4-Dihydro-2,6-dimethyl-4-(2-chlorphenyl)-5-(1,3,4-oxadiazol-2-yl)-pyridin-3-carbonsäure-(2-cyanethyl)-ester vom Fp: 180 bis 182°C.

14 g des so hergestellten 1,4-Dihydro-2,6-dimethyl-4-(2-chlorphenyl)-5-(1,3,4-oxadiazol-2-yl)-pyridin-3-carbonsäure-(2-cyanethyl)-esters werden in einer Lösung von 4,8 g NaOH in 120 ml Wasser und 60 ml Dimethoxyethan 3 h bei Raumtemperatur gerührt. Die Mischung wird mit 100 ml Wasser verdünnt und dreimal mit je 50 ml $CH_2Cl_2$ ausgeschüttelt. Die Wasser-Phase wird durch Zusatz von konzentrierter Salzsäure sauer gestellt. Danach wird der ausgefallene Niederschlag abgesaugt, mit Wasser neutral gewaschen und im Vakuum bei 50°C getrocknet. Man erhält 11,2 g (entsprechend 92,8 % d. Theorie) 1,4-Dihydro-2,6-dimethyl-4-(2-chlorphenyl)-5-(1,3,4-oxadiazol-2-yl)-pyridin-3-carbonsäure vom Schmelzpunkt 187 - 190°C.

Analyse: $C_{16}H_{13}N_3O_3Cl$ (mol 330,8)

| | | | | | |
|---|---|---|---|---|---|
| Berechnet: | C:58,09 | H:3,96 | N:12,70 | O:14,51 | Cl:10,73 |
| Gefunden: | 58,2 | 4,0 | 12,5 | 14,3 | 10,8 |

Beispiel 2:
1,4-Dihydro-2,6-dimethyl-4-(2,3-dichlorphenyl)-5-(1,3,4-oxadiazol-2-yl)-pyridin-3-carbonsäure:

28,3 g 1-(2,3-Dichlorphenyl)-2-(1,3,4-oxadiazol-2-yl)-but-1-en-3-on und 15,4 g Aminocrotonsäure-(2-cyanethyl)-ester werden in 100 ml DMF 10 h auf 80°C erwärmt. Die erkaltete Reaktionsmischung wird auf 400 ml Wasser gegossen und zweimal mit Essigester (100 ml) ausgeschüttelt. Die organische Phase wird über $Na_2SO_4$ getrocknet und eingeengt. Der Rückstand wird aus Isopropanol umkristallisiert:

33 g, (~72,7 % d. Theorie) 1,4-Dihydro-2,6-dimethyl-4-(2,3-dichlorphenyl)-5-(1,3,4-oxadiazol-2-yl)-pyridin-3-carbonsäure-2-cyanethylester vom Fp = 210 bis 212°C.

30 g des so hergestellten 1,4-Dihydro-2,6-dimethyl-4-(2,3-dichlorphenyl)-5-(1,3,4-oxadiazol-2-yl)-pyridin-3-carbonsäure-2-cyanethylesters werden in einer Lösung von 8,7 g NaOH in 220 ml Wasser und 110 ml Dimethoxyethan 3 h bei Raumtemperatur gerührt. Die Mischung wird mit 200 ml Wasser verdünnt und dreimal mit je 100 ml $CH_2Cl_2$ ausgeschüttelt. Die Wasser-Phase wird durch Zusatz von konzentrierter Salzsäure sauer gestellt. Danach wird der ausgefallene Niederschlag abgesaugt, mit Wasser neutral gewaschen und im Vakuum bei 50°C getrocknet. Man erhält 22,0 g (entsprechend 91,1 % d. Theorie) 1,4-Dihydro-2,6-dimethyl-4-(2,3-dichlorphenyl)-5-(1,3,4-oxadiazol-2-yl)-pyridin-3-carbonsäure vom Schmelzpunkt 187 - 190°C.

Analyse: $C_{16}H_{12}N_3O_3Cl_2$ (mol 365,3)

| | | | | | |
|---|---|---|---|---|---|
| Berechnet: | C:52,60 | H:3,31 | N:11,50 | O:13,14 | Cl:19,44 |
| Gefunden: | 52,4 | 3,2 | 11,3 | 13,4 | 19,5 |

Beispiel 3:
1,4-Dihydro-2,6-dimethyl-3-(1,3,4-oxadiazol-2-yl)-4-(2-chlorphenyl)-pyridin

11 g 1,4-Dihydro-2,6-dimethyl-4-(2-chlorphenyl)-5-(1,3,4-oxadiazol-2-yl)-pyridin-3-carbonsäure, hergestellt gemäß Beispiel 1, werden in 50 ml Diethylenglykol 10 min auf

180°C erwärmt. Dann wird auf Raumtemperatur abgekühlt, mit 250 ml Wasser versetzt, die Mischung viermal mit 100 ml Essigester ausgeschüttelt und die organische Phase über Natriumsulfat getrocknet und eingeengt. Der Eindampfrückstand wird aus Essigester unter Zusatz von Aktivkohle umkristallisiert.

Man erhält 5,5 g (entsprechend 57,7 % d.Theorie) 1,4-Dihydro-2,6-dimethyl-3-(1,3,4-oxadiazol-2-yl)-4-(2-chlorphenyl)-pyridin vom Schmelzpunkt 192 bis 194°C.
Analyse: $C_{15}H_{13}N_3OCl$ (mol 286,79)

| | | | | | |
|---|---|---|---|---|---|
| Berechnet: | C:62,82 | H:4,57 | N:14,65 | O:5,58 | Cl:12,38 |
| Gefunden: | 62,7 | 4,3 | 14,7 | 5,6 | 12,5 |

<u>Beispiel 4:</u>
<u>1,4-Dihydro-2,6-dimethyl-3-(1,3,4-oxadiazol-2-yl)-4-(2,3-dichlorphenyl)-pyridin</u>

20 g 1,4-Dihydro-2,6-dimethyl-4-(2,3-dichlorphenyl)-5-(1,3,4-oxadiazol-2-yl)-pyridin-3-carbonsäure werden in 100 ml Diethylenglykol 10 min auf 180°C erwärmt. Dann wird auf Raumtemperatur abgekühlt, 500 ml Wasser zugefügt und die Mischung viermal mit 200 ml Essigester ausgeschüttelt. Die organische Phase wird über Natriumsulfat getrocknet und eingeengt, der Rückstand aus Essigester unter Zusatz von Aktivkohle umkristallisiert. Man erhält 12,2 g (entsprechend 61 % d.Theorie) 1,4-Dihydro-2,6-dimethyl-3-(1,3,4-oxadiazol-2-yl)-4-(2,3-dichlorphenyl)-pyridin, Schmelzpunkt 235°C unter Zersetzung.

Analyse: $C_{15}H_{12}N_3OCl_2$ (mol 321,28)

| | | | | | |
|---|---|---|---|---|---|
| Berechnet: | C:56,08 | H:3,76 | N:13,08 | O:4,98 | Cl:22,10 |
| Gefunden: | 56,1 | 3,6 | 13,0 | 5,1 | 22,1 |

<u>Beispiele 5 bis 30</u>

Analog den Beispielen 1 bis 4 werden die folgenden Verbindungen hergestellt.

1,4-Dihydro-2,6-dimethyl-4-(3-chlorphenyl)-5-(1,3,4-oxadiazol-2-yl)-pyridin-3-carbonsäure, Fp: 182 - 184°C;
1,4-Dihydro-2,6-dimethyl-4-(3-nitrophenyl)-5-(3-methyl-1,2,4-oxadiazol-5-yl)-pyridin-3-carbonsäure, Fp: 209 - 211°C;
1,4-Dihydro-2,6-dimethyl-4-(2-nitrophenyl)-5-(3-ethyl-1,2,4-oxadiazol-5-yl)-pyridin-3-carbonsäure, Fp: 205°C;
1,4-Dihydro-2,6-dimethyl-4-(2-trifluormethyl-phenyl)-5-(1,3,4-oxadiazol-2-yl)-pyridin-3-carbonsäure, Fp: 198°C;
1,4-Dihydro-2,6-dimethyl-4-(3-trifluormethyl-phenyl)-5-(2-methyl-1,3,4-oxadiazol-5-yl)-pyridin-3-carbonsäure, Fp: 180 - 183°C;
1,4-Dihydro-2,6-dimethyl-4-(2-methoxyphenyl)-5-(5-benzyl-1,3,4-oxadiazol-2-yl)-pyridin-3-carbonsäure, Fp: 231 - 233°C;
1,4-Dihydro-2,6-dimethyl-4-(3-cyanophenyl)-5-(4-methyl-5-ethoxycarbonyl-thiazol-2-yl)-pyridin-3-carbonsäure; Fp: 242 - 245°C;
1,4-Dihydro-2,6-dimethyl-4-(pyrid-2-yl)-5-(5-methoxymethyl-1,2,4-oxadiazol-3-yl)-pyridin-3-carbonsäure, Fp: 198 - 201°C;
1,4-Dihydro-2,6-dimethyl-4-(2,3-dichlorphenyl)-5-(3-benzyl-1,2,4-oxadiazol-5-yl)-pyridin-3-carbonsäure, Fp: 212°C;
1,4-Dihydro-2,6-dimethyl-4-(2-thienyl)-5-(5-isopropylthio-1,3,4-oxadiazol-2-yl)-pyridin-3-carbonsäure, Fp: 217 - 218°C;
1,4-Dihydro-2,6-dimethyl-4-(2-methylphenyl)-5-(5-cyclohexyl-1,2,4-oxadiazol-3-yl)-pyridin-3-carbonsäure, Fp: 218°C;
1,4-Dihydro-2,6-dimethyl-4-(2-benzyloxyphenyl)-5-(2-ethyl-1,3,4-oxadiazol-5-yl)-pyridin-3-carbonsäure, Fp: 193 - 194°C;
1,4-Dihydro-2,6-dimethyl-4-(3-methoxyphenyl)-5-(3-methyl-1,2,4-thiadiazol-5-yl)-pyridin-3-carbonsäure, Fp: 184 - 186°C;
1,4-Dihydro-2,6-dimethyl-4-(3-chlorphenyl)-5-(1,3,4-oxadiazolyl-2-)-pyridin, Fp: 182°C;
1,4-Dihydro-2,6-dimethyl-4-(3-nitrophenyl)-5-(3-methyl-1,2,4-oxadiazol-5-yl)-pyridin, Fp: 176 - 178°C;
1,4-Dihydro-2,6-dimethyl-4-(2-nitrophenyl)-5-(3-ethyl-1,2,4-oxadiazol-5-yl)-pyridin, Fp: 209 - 211°C;
1,4-Dihydro-2,6-dimethyl-4-(2-trifluormethyl-phenyl)-5-(1,3,4-oxadiazol-2-yl)-pyridin, Fp: 199 - 202°C;
1,4-Dihydro-2,6-dimethyl-4-(3-trifluormethyl-phenyl)-5-(2-methyl-1,3,4-oxadiazol-5-yl)-pyridin, Fp: 191 - 192°C;
1,4-Dihydro-2,6-dimethyl-4-(2-methoxyphenyl)-5-(5-benzyl-1,3,4-oxadiazol-2-yl)-pyridin, Fp: 205°C;
1,4-Dihydro-2,6-dimethyl-4-(3-cyanophenyl)-5-(4-methyl-5-ethoxycarbonyl-thiazol-2-yl)-pyridin, Fp: 198 - 201°C;
1,4-Dihydro-2,6-dimethyl-4-(pyrid-2-yl)-5-(5-methoxymethyl-1,2,4-oxadiazol-3-yl)-pyridin, Fp: 188 - 189°C;
1,4-Dihydro-2,6-dimethyl-4-(2,3-dichlorphenyl)-5-(3-benzyl-1,2,4-oxadiazol-5-yl)-pyridin, Fp: 201 - 203°C;
1,4-Dihydro-2,6-dimethyl-4-(2-thienyl)-5-(5-isopropylthio-1,3,4-oxadiazol-2-yl)-pyridin, Fp: 192°C;
1,4-Dihydro-2,6-dimethyl-4-(2-methylphenyl)-5-(5-cyclohexyl-1,2,4-oxadiazol-3-yl)-pyridin, Fp: 221°C;
1,4-Dihydro-2,6-dimethyl-4-(2-benzyloxyphenyl)-5-(2-ethyl-1,3,4-oxadiazol-5-yl)-pyridin, Fp: 202 - 205°C;
1,4-Dihydro-2,6-dimethyl-4-(3-methoxyphenyl)-5-(3-methyl-1,2,4-thiadiazol-5-yl)-pyridin. Fp: 196 - 198°C;

Die folgenden Beispiele A bis C betreffen pharmazeutische Zubereitungen.

Beispiel A: Dragees

| | |
|---|---|
| Wirkstoff | 10 mg |
| Milchzucker | 80 mg |
| Maisstärke | 110 mg |
| sek.Calciumphosphat | 40 mg |
| lösliche Stärke | 3 mg |
| Magnesiumstearat | 3 mg |
| kolloidale Kieselsäure | 4 mg |
| | 250 mg |

Beispiel B: Tabletten

| | |
|---|---|
| Wirkstoff | 20 mg |
| Milchzucker | 60 mg |
| Maisstärke | 35 mg |
| lösliche Stärke | 4 mg |
| Magnesiumstearat | 1 mg |
| | 120 mg |

Beispiel C: Kapseln

| | |
|---|---|
| Wirkstoff | 15 mg |
| Maisstärke | 185 mg |
| | 200 mg |

Patentansprüche

1. 1,4-Dihydropyridine der Formel I

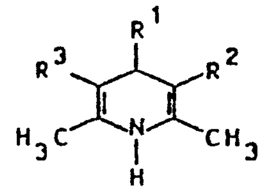

(I)

worin

$R^1$ Phenyl, Pyridyl oder Thienyl,

$R^2$ Oxadiazolyl, Thiadiazolyl oder Thiazolyl und

$R^3$ Wasserstoff oder -COOH bedeutet,

wobei ein für $R^1$ stehender Phenyl-, Pyridyl- oder Thienyl-Rest unsubstituiert ist oder einen oder zwei gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyan, Benzyloxy aufweist, ein für $R^2$ stehender Oxadiazolyl-, Thiadiazolyl- oder Thiazolyl-Rest unsubstituiert ist oder einen, oder im Falle von Thiazolyl einen oder zwei gleiche oder verschiedene, Substituenten aus der Gruppe Alkyl mit 1 bis 4 C-Atomen, Alkoxy mit 1 bis 4 C-Atomen, Alkylthio mit 1 bis 4 C-Atomen, Aralkyl mit 7 bis 9 C-Atomen, Alkoxyalkyl mit insgesamt 2 bis 5 C-Atomen, Cycloalkyl mit 5 oder 6 C-Atomen, Aminocarbonyl-methylthio, Methoxycarbonyl, Ethoxycarbonyl oder Phenyl aufweist, und deren Salze mit pharmakologisch akzeptablen Säuren.

2. 1,4-Dihydropyridine gemäß Anspruch 1 der Formel I, dadurch gekennzeichnet, daß $R^1$ Phenyl bedeutet, das unsubstituiert ist oder das einen oder zwei gleiche oder verschiedene Substituenten aus der Reihe Halogen, Methyl, Methoxy, Nitro, Trifluormethyl, Cyan oder Benzyloxy aufweist.

3. 1,4-Dihydropyridine gemäß Anspruch 1 der Formel I, dadurch gekennzeichnet, daß $R^2$ einen 1,2,4- oder 1,3,4-Oxadiazolylrest bedeutet, der einen Substituenten aus der Reihe Methyl, Ethyl, Isopropyl oder Benzyl aufweist.

4. 1,4-Dihydro-2,6-dimethyl-3-(1,3,4-oxadiazol-2-yl)-4-(2-chlorphenyl)-pyridin.

5. 1,4-Dihydro-2,6-dimethyl-3-(3-ethyl-1,2,4-oxadiazol-5-yl)-4-(2-trifluormethyl-phenyl)-pyridin.

6. 1,4-Dihydro-2,6-dimethyl-3-(1,3,4-oxadiazol-2-yl)-4-(2,3-dichlorphenyl)-pyridin.

7. Verfahren zur Herstellung von 1,4-Dihydropyridinen der Formel I

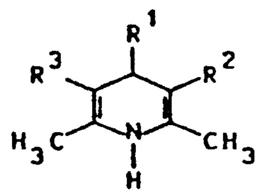

(I)

worin
$R^1$ Phenyl, Pyridyl oder Thienyl,
$R^2$ Oxadiazolyl, Thiadiazolyl oder Thiazolyl und
$R^3$ Wasserstoff oder -COOH bedeutet,
wobei ein für $R^1$ stehender Phenyl-, Pyridyl- oder Thienyl-Rest unsubstituiert ist oder einen oder zwei gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyan, Benzyloxy aufweist, ein für $R^2$ stehender Oxadiazolyl-, Thiadiazolyl- oder Thiazolyl-Rest unsubstituiert ist oder einen, oder im Falle von Thiazolyl einen oder zwei gleiche oder verschiedene, Substituenten aus der Gruppe Alkyl mit 1 bis 4 C-Atomen,

Alkoxy mit 1 bis 4 C-Atomen, Alkylthio mit 1 bis 4 C-Atomen, Aralkyl mit 7 bis 9 C-Atomen, Alkoxyalkyl mit insgesamt 2 bis 5 C-Atomen, Cycloalkyl mit 5 oder 6 C-Atomen, Aminocarbonyl-methylthio, Methoxycarbonyl, Ethoxycarbonyl oder Phenyl aufweist, und deren Salze mit pharmakologisch akzeptablen Säuren, dadurch gekennzeichnet, daß man einen Aminocrotonsäureester der Formel II

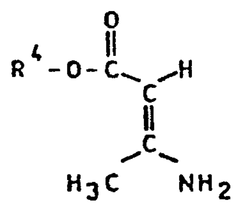

(II)

worin $R^4$ der Rest eines hydrolytisch leicht abspaltbaren Alkohols ist und vorzugsweise β-Cyanethyl bedeutet, mit einem α,β-ungesättigten Keton der Formel III

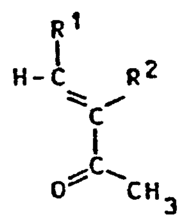

(III)

worin $R^1$ und $R^2$ die oben genannten Bedeutungen haben, in Wasser oder einem inerten organischen Lösungsmittel bei einer Temperatur zwischen 10°C und dem Siedepunkt des eingesetzten Lösungsmittels zu einem 1,4-Dihydropyridincarbonsäureester der Formel Ia

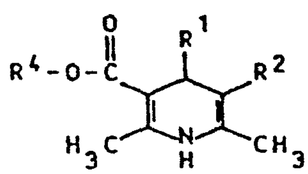

(Ia)

worin $R^1$ bis $R^4$ die oben genannten Bedeutungen haben, umsetzt, anschließend diesen Ester in wäßrigem Medium in Gegenwart einer Säure oder vorzugsweise einer Base zu einer Verbindung der Formel I, in der $R^3$ die Carboxylgruppe ist, hydrolisiert und gewünschtenfalls die erhaltene Verbindung in an sich bekannter Weise decarboxyliert und gegebenenfalls mit einer pharmakologisch akzeptablen Säure zu dem entsprechenden Säureadditionssalz umsetzt.

8. Verfahren gemäß Anspruch 7 zur Herstellung von 1,4-Dihydropyridinen der Formel I, dadurch gekennzeichnet, daß man die Aminocarbonsäureester der Formel II mit einem Aldehyd der Formel IV

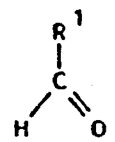 (IV)

worin $R^1$ die oben genannte Bedeutung hat, und einem Keton der Formel V

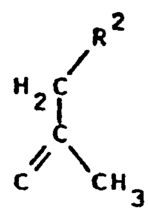 (V)

worin $R^2$ die oben genannte Bedeutung hat, im Molverhältnis 1 : 1 : 1 in Wasser oder einem inerten organischen Lösungsmittel bei einer Temperatur zwischen 10 °C und dem Siedepunkt des eingesetzten Lösungsmittels zu einem 1,4-Dihydropyridin-carbonsäureester der Formel Ia, worin $R^1$ bis $R^4$ die oben genannten Bedeutungen haben, umsetzt und anschließend gemäß Anspruch 7 hydrolysiert und gegebenenfalls decarboxyliert und in ein Säureadditionssalz überführt.

9. Pharmazeutische Zubereitung, enthaltend eine pharmakologisch wirksame Dosis eines 1,4-Dihydropyridins der Formel I einer oder mehrerer der Ansprüche 1 bis 6.

10. Verwendung von 1,4-Dihydropyridinen der Formel I eines oder mehrerer der Ansprüche 1 bis 6 als Calciumagonist in der Humantherapie.

PATENTANSPRÜCHE für den Vertragsstaat Österreich

1. Verfahren zur Herstellung von 1,4-Dihydropyridinen der Formel I

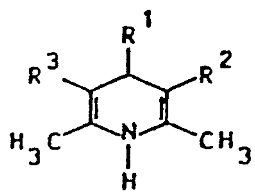

(I)

worin
$R^1$ Phenyl, Pyridyl oder Thienyl,
$R^2$ Oxadiazolyl, Thiadiazolyl oder Thiazolyl und
$R^3$ Wasserstoff oder -COOH bedeutet,
wobei ein für $R^1$ stehender Phenyl-, Pyridyl- oder Thienyl-Rest unsubstituiert ist oder einen oder zwei gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyan, Benzyloxy aufweist, ein für $R^2$ stehender Oxadiazolyl-, Thiadiazolyl- oder Thiazolyl-Rest unsubstituiert ist oder einen, oder im Falle von Thiazolyl einen oder zwei gleiche oder verschiedene, Substituenten aus der Gruppe Alkyl mit 1 bis 4 C-Atomen, Alkoxy mit 1 bis 4 C-Atomen, Alkylthio mit 1 bis 4 C-Atomen, Aralkyl mit 7 bis 9 C-Atomen, Alkoxyalkyl mit insgesamt 2 bis 5 C-Atomen, Cycloalkyl mit 5 oder 6 C-Atomen, Aminocarbonyl-methylthio, Methoxycarbonyl, Ethoxycarbonyl oder Phenyl aufweist, und deren Salze mit pharmakologisch akzeptablen Säuren, dadurch gekennzeichnet, daß man einen Aminocrotonsäureester der Formel II

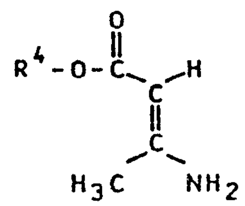 (II)

worin $R^4$ der Rest eines hydrolytisch leicht abspaltbaren Alkohols ist, mit einem α,β-ungesättigten Keton der Formel III

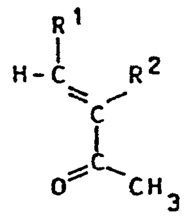 (III)

worin $R^1$ und $R^2$ die oben genannten Bedeutungen haben, in Wasser oder einem inerten organischen Lösungsmittel bei einer Temperatur zwischen $10^oC$ und dem Siedepunkt des eingesetzten Lösungsmittels zu einem 1,4-Dihydropyridincarbonsäureester der Formel Ia

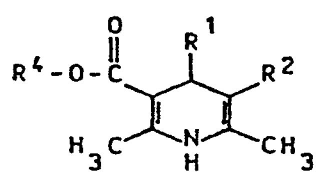 (Ia)

worin $R^1$ bis $R^4$ die oben genannten Bedeutungen haben, umsetzt, anschließend diesen Ester in wäßrigem Medium in Gegenwart einer Säure oder vorzugsweise einer Base zu einer Verbindung der Formel I, in der $R^3$ die Carboxylgruppe ist, hydrolisiert und gewünschtenfalls die erhaltene Verbindung in an sich bekannter Weise decarboxyliert

und gegebenenfalls mit einer pharmakologisch akzeptablen Säure zu dem entsprechenden Säureadditionssalz umsetzt.

2. Verfahren gemäß Anspruch 1 zur Herstellung von 1,4-Dihydropyridinen der Formel I, dadurch gekennzeichnet, daß man die Aminocarbonsäureester der Formel II mit einem Aldehyd der Formel IV

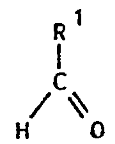 (IV)

worin $R^1$ die oben genannte Bedeutung hat, und einem Keton der Formel V

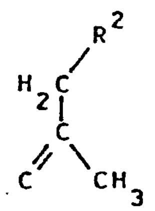 (V)

worin $R^2$ die oben genannte Bedeutung hat, im Molverhältnis 1 : 1 : 1 in Wasser oder einem inerten organischen Lösungsmittel bei einer Temperatur zwischen 10 °C und dem Siedepunkt des eingesetzten Lösungsmittels zu einem 1,4-Dihydropyridin-carbonsäureester der Formel Ia, worin $R^1$ bis $R^4$ die oben genannten Bedeutungen haben, umsetzt und anschließend gemäß Anspruch 7 hydrolysiert und gegebenenfalls decarboxyliert und in ein Säureadditionssalz überführt.

3. Verfahren nach Anspruch 1 und/oder 2, dadurch gekennzeichnet, daß ein Aminocrotonsäureester der Formel II eingesetzt wird, worin $R^4$ β-Cyanethyl bedeutet.

4. Verfahren nach einem oder mehreren der Ansprüche 1 bis 3, dadurch gekennzeichnet, daß die Ausgangsverbindungen

so ausgewählt werden, daß Verbindungen der Formel I entstehen, wobei $R^1$ Phenyl bedeutet, das unsubstituiert ist oder das einen oder zwei gleiche oder verschiedene Substituenten aus der Reihe Halogen, Methyl, Methoxy, Nitro, Trifluormethyl, Cyan oder Benzyloxy aufweist.

5. Verfahren nach einem oder mehreren der Ansprüche 1 bis 4, dadurch gekennzeichnet, daß die Ausgangsverbindungen so ausgewählt werden, daß Verbindungen der Formel I entstehen, wobei $R^2$ einen 1,2,4- oder 1,3,4-Oxadiazolylrest bedeutet, der einen Substituenten aus der Reihe Methyl, Ethyl, Isopropyl oder Benzyl aufweist.

6. Verfahren nach einem oder mehreren der Ansprüche 1 bis 5, dadurch gekennzeichnet, daß die Ausgangsverbindungen so ausgewählt werden, daß die Verbindung 1,4-Dihydro-2,6-dimethyl-3-(1,3,4-oxadiazol-2-yl)-4-(2-chlorphenyl)-pyridin entsteht.

7. Verfahren nach einem oder mehreren der Ansprüche 1 bis 5, dadurch gekennzeichnet, daß die Ausgangsverbindungen so ausgewählt werden, daß die Verbindung 1,4-Dihydro-2,6-dimethyl-3-(3-ethyl-1,2,4-oxadiazol-5-yl)-4-(2-trifluormethyl-phenyl)-pyridin entsteht.

8. Verfahren nach einem oder mehreren der Ansprüche 1 bis 5, dadurch gekennzeichnet, daß die Ausgangsverbindungen so ausgewählt werden, daß die Verbindung 1,4-Dihydro-2,6-dimethyl-3-(1,3,4-oxadiazol-2-yl)-4-(2,3-dichlorphenyl)-pyridin entsteht.

9. Verwendung eines 1,4-Dihydropyridins der Formel I

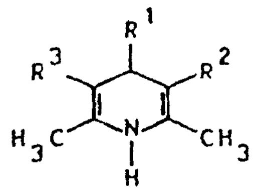

(I)

worin

$R^1$ Phenyl, Pyridyl oder Thienyl,

$R^2$ Oxadiazolyl, Thiadiazolyl oder Thiazolyl und

$R^3$ Wasserstoff oder -COOH bedeutet,

wobei ein für $R^1$ stehender Phenyl-, Pyridyl- oder Thienyl-Rest unsubstituiert ist oder einen oder zwei gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyan, Benzyloxy aufweist, ein für $R^2$ stehender Oxadiazolyl-, Thiadiazolyl- oder Thiazolyl-Rest unsubstituiert ist oder einen, oder im Falle von Thiazolyl einen oder zwei gleiche oder verschiedene, Substituenten aus der Gruppe Alkyl mit 1 bis 4 C-Atomen, Alkoxy mit 1 bis 4 C-Atomen, Alkylthio mit 1 bis 4 C-Atomen, Aralkyl mit 7 bis 9 C-Atomen, Alkoxyalkyl mit insgesamt 2 bis 5 C-Atomen, Cycloalkyl mit 5 oder 6 C-Atomen, Aminocarbonyl-methylthio, Methoxycarbonyl, Ethoxycarbonyl oder Phenyl aufweist, oder eines pharmakologisch akzeptablen Säureadditionssalzes davon zur Herstellung calciumantagonistisch wirkender Mittel.